# DEMANDE DE BREVET EUROPEEN

(11) **EP 1 468 980 A1**
(43) Date de publication de la demande: **20.10.2004**
(21) Numéro de dépôt: 04290802.0
(22) Date de dépôt: 25.03.2004
(51) Int. Cl.: C07C 51/56, C07C 53/124

(54) **Procédé de fabrication de l'anhydride isobutyrique**

(30) Priorité: 16.04.2003 FR 0304785
(71) Demandeur: Atofina, 92800 Puteaux (FR)
(72) Inventeur: Paul, Jean-Michel, 57070 Metz (FR); Busca, Patrick, 57800 Bening Les Saint-Avold (FR)

(57) **Abrégé**

Ce procédé de fabrication de l'anhydride isobutyrique par réaction de l'anhydride acétique avec l'acide isobutyrique, en distillant l'acide acétique généré au fur et à mesure de sa formation, est caractérisé par le fait que l'on charge le réacteur au départ avec au moins une partie de l'un des réactifs et une partie de l'autre de telle sorte que les réactifs soient dans un rapport molaire excédentaire par rapport à la stoechiométrie en l'un des réactifs, et on fait réagir tout en ajoutant le complément des réactifs au fur et à mesure de l'avancement de la réaction et en fonction de la place laissée libre dans le réacteur par la distillation de l'acide acétique produit par la réaction, jusqu'à atteindre le rapport molaire global des réactifs souhaité.

## Description

La présente invention concerne un procédé de fabrication de l'anhydride isobutyrique.

On sait depuis longtemps qu'il est possible de préparer un anhydride d'acide carboxylique par réaction d'anhydride acétique avec un acide carboxylique correspondant à l'anhydride désiré.

Le brevet français FR-B-784 458 décrit la préparation des anhydrides propanoïque, butyrique et caproïque par réaction entre l'anhydride acétique et les acides respectivement propanoïque, butyrique ou caproïque, en distillant l'acide acétique généré au fur et à mesure de sa formation.

La demande de brevet européen n° EP-A-4 641 décrit un procédé continu ou discontinu pour la préparation d'anhydrides carboxyliques, tels que les anhydrides benzoïque, hexahydrobenzoïque et triméthylacétique, par réaction entre les acides carboxyliques et l'anhydride acétique, de préférence dans les proportions stoechiométriques.

Le brevet français FR-B-2 514 345 décrit la fabrication d'anhydride isobutyrique à partir de propylène par une carboxylation dans le fluorure d'hydrogène liquide, suivie d'une hydrolyse partielle du fluorure d'isobutyryle. Ce procédé est difficile à mettre en oeuvre compte tenu de la nature des produits en jeu.

La présente invention a donc pour objectif de proposer un procédé de fabrication de l'anhydride isobutyrique beaucoup plus simple dans sa mise en oeuvre que le procédé connu selon FR-B-2 514 345 et qui permet d'obtenir un anhydride isobutyrique d'excellente pureté dans des conditions améliorées vis-à-vis du procédé selon FR-B-784 458.

La présente invention a pour objet un procédé de fabrication de l'anhydride isobutyrique par réaction de l'anhydride acétique avec l'acide isobutyrique, en distillant l'acide acétique généré au fur et à mesure de sa formation, caractérisé par le fait que l'on charge le réacteur au départ avec au moins une partie de l'un des réactifs et une partie de l'autre de telle sorte que les réactifs soient dans un rapport molaire excédentaire par rapport à la stoechiométrie en l'un des réactifs, et on fait réagir tout en ajoutant le complément des réactifs au fur et à mesure de l'avancement de la réaction et en fonction de la place laissée libre dans le réacteur par la distillation de l'acide acétique produit par la réaction, jusqu'à atteindre le rapport molaire global des réactifs souhaité.

Est ainsi utilisée utilise la technique dite « d'addition différée » dans une réaction en discontinu (« batch ») avec déplacement des divers équilibres mis en jeu entre les espèces présentes. Une telle technique a permis d'obtenir de façon surprenante une production notablement accrue d'anhydride isobutyrique.

Avantageusement, la réaction est conduite sans ajout de catalyseur.

Conformément à un mode de réalisation particulier du procédé selon la présente invention, on charge au départ la totalité de l'un des réactifs et une partie du second.

Le rapport molaire acide isobutyrique / anhydride acétique global (après addition du ou des réactifs chargés partiellement au départ) peut être compris entre 0,5 et 5, en particulier entre 1,5 et 2,2.

Le rapport molaire initial acide isobutyrique / anhydride acétique ou anhydride acétique / acide isobutyrique peut avantageusement être compris entre 0,2 et 1.

On conduit avantageusement la réaction dans un réacteur agité surmonté d'une colonne à distiller dont l'efficacité est de préférence d'au moins 8 plateaux théoriques. En effet, la colonne doit être suffisamment efficace pour éviter de monter de l'anhydride acétique avec l'acide acétique, la pureté de l'acide acétique distillé étant dépendante de l'efficacité de la colonne.

Le réacteur est avantageusement chauffé par circulation d'un fluide caloporteur dans une double enveloppe ou par recirculation au travers d'un échangeur de chaleur.

Le garnissage de la colonne peut être un garnissage en vrac classique ou structuré ou un mélange vrac/structuré.

On conduit la réaction généralement à une température de 70 à 150°C, de préférence de 100 à 120°C. Des températures inférieures à 70°C sont possibles mais au détriment de la cinétique de la réaction.

Le maintien de la température à la valeur de consigne est réalisé par l'ajustement de la pression dans le réacteur.

On conduit la réaction généralement à une pression comprise entre 5,33 x 10⁴ Pa (400 mmHg) et 0,67 x 10⁴ Pa (50 mmHg).

Avantageusement, on ajuste la consigne de température en tête de colonne en fonction de la pression afin de correspondre à la température de distillation de l'acide acétique durant toute la réaction.

Après l'achèvement de la réaction, on peut purifier le brut en distillant l'excès d'anhydride acétique et l'anhydride mixte résiduel.

L'anhydride isobutyrique ainsi préparé a une pureté supérieure à 98%. Une pureté de plus de 99% peut être obtenue en distillant le brut.

### EXEMPLES :

Les exemples suivants illustrent la présente invention sans toutefois en limiter la portée. Dans ces exemples, les pourcentages sont des pourcentages massiques.

### EXEMPLE 1 (comparatif) :

Dans un réacteur agité mécaniquement (agitateur de type ancre), chauffé par circulation d'un fluide caloporteur dans une double enveloppe, et surmonté d'une colonne à distiller à garnissage Multiknit ®, d'efficacité égale à 9 plateaux théoriques, avec condenseur en tête, séparateur à vide, recette et piège, on introduit en une fois :
- 232,3 g (2,28 moles) d'anhydride acétique ;
- 320,6 g d'acide isobutyrique (3,64 moles)
soit dans un rapport molaire acide isobutyrique/anhydride acétique de 1,6.

La charge totale initiale est de 552,9 g.

On maintient une température comprise entre 115 et 120°C pendant toute la réaction en baissant progressivement la pression de 4,00 x 10⁴ Pa (300 mmHg) à 1,07 x 10⁴ Pa (80 mmHg).

L'acide acétique produit à la réaction est distillé au fur et à mesure de sa formation. On obtient ainsi une première fraction de distillation (F1) de 220 g (pureté : 96,1%).

L'excès d'anhydride acétique et le mixte résiduel sont éliminés par distillation à une pression de 1,07 x 10⁴ Pa (80 mmHg) (fraction F2 : 71 g).

On obtient un brut de pied (263 g) ayant une pureté de 98,7%.

Si nécessaire, on peut obtenir un anhydride isobutyrique de pureté supérieure à 99% par distillation.

### EXEMPLE 2 (de l'invention) :

Le réacteur décrit à l'Exemple 1 est chargé avec la totalité de l'anhydride acétique mis en oeuvre (411 g) et une partie de l'acide isobutyrique (141,9 g), soit avec un rapport molaire initial acide isobutyrique / anhydride acétique égal à 0,4.

La charge totale initiale est de 552,9 g.

Pendant la phase réactionnelle, au fur et à mesure de la distillation de l'acide acétique, on ajoute en continu à un débit calé sur celui de l'acide acétique distillé, 425,6 g d'acide isobutyrique de telle manière que le rapport molaire global final acide isobutyrique / anhydride acétique soit égal à 1,6.

La température de réaction est maintenue entre 115 et 120°C en baissant progressivement la pression de 5,33 x 10⁴ Pa (400 mmHg) à 1,60 x 10⁴ Pa (120 mmHg).

L'acide acétique produit à la réaction est éliminé par distillation au fur et à mesure de sa formation. On obtient ainsi une première fraction de distillation (F1) de 415 g, de pureté en acide acétique égale à 96 %.

Le reste d'acide acétique, l'excès d'anhydride acétique, ainsi que l'anhydride mixte résiduel sont éliminés par distillation à 1,07 x 10⁴ Pa (80 mmHg) (fraction F2 : 75 g).

Le brut ainsi étêté (479 g) a une pureté de 98,3%.

Une pureté de 99% peut être obtenue en distillant ce brut.

Le gain de production pour une même charge initiale de réactifs est d'environ 80% sans augmentation importante de la durée de réaction.

## Revendications

1. Procédé de fabrication de l'anhydride isobutyrique par réaction de l'anhydride acétique avec l'acide isobutyrique, en distillant l'acide acétique généré au fur et à mesure de sa formation, **caractérisé par le fait que** l'on charge le réacteur au départ avec au moins une partie de l'un des réactifs et une partie de l'autre de telle sorte que les réactifs soient dans un rapport molaire excédentaire par rapport à la stoechiométrie en l'un des réactifs, et on fait réagir tout en ajoutant le complément des réactifs au fur et à mesure de l'avancement de la réaction et en fonction de la place laissée libre dans le réacteur par la distillation de l'acide acétique produit par la réaction, jusqu'à atteindre le rapport molaire global des réactifs souhaité.

2. Procédé selon la revendication 1, **caractérisé par le fait que** l'on charge au départ la totalité de l'un des réactifs et une partie du second.

3. Procédé selon l'une des revendications 1 et 2, **caractérisé par le fait que** le rapport molaire global acide isobutyrique / anhydride acétique est compris entre 0,5 et 5.

4. Procédé selon la revendication 3, **caractérisé par le fait que** le rapport molaire global acide isobutyrique / anhydride acétique est compris entre 1,5 et 2,2.

5. Procédé selon l'une des revendications 1 à 4, **caractérisé par le fait que** le rapport molaire initial acide isobutyrique / anhydride acétique ou anhydride acétique / acide isobutyrique est compris entre 0,2 et 1.

6. Procédé selon l'une des revendications 1 à 5, **caractérisé par le fait que** l'on conduit la réaction dans un réacteur agité surmonté d'une colonne à distiller dont l'efficacité est d'au moins 8 plateaux théoriques.

7. Procédé selon l'une des revendications 1 à 6, **caractérisé par le fait que** l'on conduit la réaction à une température de 70 à 150°C, de préférence de 100 à 120°C.

8. Procédé selon l'une des revendications 1 à 7, **caractérisé par le fait que** l'on conduit la réaction à une pression comprise entre 5,33 x 10⁴ Pa (400 mmHg) et 0,67 x 10⁴ Pa (50 mmHg) .

9. Procédé selon l'une des revendications 1 à 8, **caractérisé par le fait que** l'on ajuste la consigne de température en tête de colonne en fonction de la pression afin de correspondre à la température de distillation de l'acide acétique durant toute la réaction.

10. Procédé selon l'une des revendications 1 à 9, **caractérisé par le fait qu'**après l'achèvement de la réaction, on purifie le brut en distillant l'excès d'anhydride acétique et l'anhydride mixte résiduel.
